## Europäisches Patentamt
## European Patent Office
## Office européen des brevets

(19)

(11) Veröffentlichungsnummer: **0 271 447 B1**

(12) **EUROPÄISCHE PATENTSCHRIFT**

(45) Veröffentlichungstag der Patentschrift: **10.03.93**

(51) Int. Cl.⁵: **A61K 37/66**

(21) Anmeldenummer: **87810723.4**

(22) Anmeldetag: **07.12.87**

(54) **Vorbeugende Massnahmen zur Verhinderung der "Komplexen Respiratorischen Erkrankung bei Rindern" durch Verabreichung von Interferon.**

(30) Priorität: **12.12.86 US 941453**
**13.11.87 US 117840**

(43) Veröffentlichungstag der Anmeldung:
**15.06.88 Patentblatt 88/24**

(45) Bekanntmachung des Hinweises auf die
Patenterteilung:
**10.03.93 Patentblatt 93/10**

(84) Benannte Vertragsstaaten:
**AT BE CH DE ES FR GB GR IT LI LU NL SE**

(73) Patentinhaber: **CIBA-GEIGY AG**
**Klybeckstrasse 141**
**CH-4002 Basel(CH)**

(72) Erfinder: **Anderson, Kevin**
**827 Buena Vista**
**Moss Beach California 94038(US)**
Erfinder: **Babiuk, Lorne**
**15 Murphy Crescent**
**Saskatoon Saskatchewan SJ7 2T5(CA)**
Erfinder: **McCracken, Joseph Stephen**
**3814 Naughton Avenue**
**Belmont California 94002(US)**
Erfinder: **Ohmann, Helle Bielefeldt**
**502, 5th Avenue North**
**Saskatoon Saskatchewan S7K 2R2(CA)**
Erfinder: **Philip, John**
**3025 Pleasant Ridge Road**
**Summerfield North Carolina 27358(US)**

Rank Xerox (UK) Business Services
(3.10/3.5x/3.0.1)

**Beschreibung**

Der Gegenstand der Erfindung betrifft das Gebiet der Tiergesundheit, wobei die Bereitstellung eines Arzneimittels zur Eindämmung der auf multifaktoriellen Ursachen beruhenden "Komplexen Respiratorischen Erkrankung bei Rindern" im Vordergrund steht.

Es sind inzwischen verschiedene Formen von respiratorischen Erkrankungen bei Rindern bekannt, die im allgemeinen unter dem Sammelbegriff der "Komplexen Respiratorischen Erkrankung bei Rindern" ["Rindergrippe Komplex"; "bovine respiratory disease complex" (BRD)] zusammengefasst werden.

Diese rinderspezifische Erkrankung der Atmungsorgane ist eine der Hauptursachen für die Verluste der Rinder-Industrie.

Eine Form dieser Erkrankung (BRD), das sogenannte "Shipping Fever" tritt in erster Linie bei frisch entwöhnten Kälbern auf, und zwar in der Regel bereits innerhalb der ersten Woche, nachdem die Kälber in Maststationen verbracht worden sind, da sie aufgrund der Behandlung, des Transports sowie gegebenenfalls ungünstiger Witterungseinflüsse einem starken Stress ausgesetzt sind.

Beim "Shipping Fever" handelt es sich um eine der am weitesten verbreiteten und unter ökonomischen Gesichtspunkten signifikantesten Erkrankungen bei Rindern, die der Nordamerikanischen Rinder-Industrie jährliche Verluste zwischen 250 Millionen und 1 Milliarde US-Dollars verursacht.

Eine zweite Form der "BRD" betrifft die "Enzootische Kälber-Pneumonie", die vornehmlich bei Kälbern auftritt, die in geschlossenen Stallungen gehalten werden, und zwar in der Regel innerhalb des ersten Lebensmonats.

Die davon betroffenen Kälber entwickeln dabei akute respiratorische Krankheitssymptome, die schliesslich zum Tode der Tiere führen können.

Andere Tiere dagegen zeigen einen milderen Krankheitsverlauf, der mit Irritationen der Nasen- und Augenschleimhäute verbunden ist.

Die betroffenen Kälber können im weiteren Verlauf der Krankheit entweder ebenfalls akutere Krankheitssymptome entwickeln oder aber sie erholen sich wieder, wobei dann jedoch ein zweiter Krankheitsausbruch innerhalb eines Zeitraumes von mehreren Wochen möglich ist.

Obwohl diese Erkrankung am häufigsten bei Rindern anzutreffen ist, die für die Fleischproduktion bestimmt sind, findet man sie dennoch auch beim Milchvieh. Die Erkrankung erreicht alle Altersstadien, wobei jedoch in erster Linie frisch eingekauftes junges Vieh, das neu in eine Herde integriert worden ist, betroffen ist. In manchen Fällen kann die Eingliederung von neuem Milchvieh in eine bestehende Herde sogar den Ausbruch einer Pneumonie im eigenen Bestand zur Folge haben.

Die unter dem Begriff des Rinder-Grippe-Komplexes zusammengefassten Erkrankungen werden vermutlich von einer Reihe von Faktoren ausgelöst, wie beispielsweise durch Schädigung des respiratorischen Traktes und/oder durch Stress. Eine Schädigung des respiratorischen Traktes kann beispielsweise durch Viren ausgelöst werden, wie das Rinder-Herpes-Virus, sowie durch verschiedene Mycoplasma und/oder Chlamydia-Stämme. Stress, kann z.B. durch Entwöhnung, Kastration oder zu hohe Individuendichte, durch Temperaturänderungen sowie durch sekundäre Infektionen durch Bakterien, wie z.B. verschiedene Pasteurella-Arten verursacht werden.

Eine Kombination von Virusbefall und Stresseinwirkung kann bei den betroffenen Rindern zu einer Immunsuppression führen. Diese Immunsuppression kann in der Folge eine sekundäre Kolonisierung der Lungen mit Bakterien (in erster Linie mit Pasteurella haemolytica) (siehe Joseph M. Cummings, "Feedlot Disease and Parasites" in The Feedlot, G.B. Thompson and Clayton C. O'Mary Ed., Lea and Febiger Philadelphia, Third Ed., 1983) begünstigen.

Prädisponierende Faktoren sind somit für die Anfälligkeit gegenüber "Shipping Fever" oder der "Enzootischen Kälberpneumonie" von entscheidender Bedeutung. In erster Linie sind hier physikalische Stress-Faktoren, chemische Agenzien sowie psychologische Faktoren zu nennen.

Die physikalischen Stress-Faktoren umfassen Entwöhnungsstress, extreme Temperaturen, plötzliche Witterungs-Veränderungen, Feuchtigkeit, unregelmässiges Füttern und Tränken der Tiere, Änderungen in den Futte-Rationen, Staub, Überfüllung sowie das Zusammenziehen und Einsperren der Tiere.

Der Stress, dem Kälber, die gerade frisch in Viehhöfen oder Maststationen eingetroffen sind, durch die sich anschliessenden Behandlungen ausgesetzt sind, wird als eine der Hauptursachen für den Ausbruch einer Pneumonie angesehen. Die Kälber werden in der Regel bereits unmittelbar nach ihrer Ankunft geimpft, enthornt, kastriert und mit Vitaminen und Antibiotika behandelt. Diese Massnahmen werden sehr oft an Rindern vorgenommen, die aufgrund des Transports in Viehwaggons unter beengten Bedingungen stark ermüdet sind.

Zu den chemischen Agenzien, die Stress auslösen können, zählen Ammoniakdämpfe und toxische Gase, die innerhalb der landwirtschaftlichen Betriebe entstehen können.

Es hat sich darüberhinaus gezeigt, dass zusätzliche Erkrankungen, wie Parasitenbefall, ebenfalls die Abwehrkräfte gegenüber Infektionen negativ beeinflussen.

All diese Faktoren führen zu pathologischen Veränderungen wie beispielsweise zu einer Depression des Immunsystems (z.B. endogener Steroid-Freisetzung, die die Funktionsfähigkeit der Neutrophilen und Lymphozyten einschränkt) sowie Paralyse oder Hemmung der Mucin-Sekretion im Atmungstrakt.

Zu den bekanntesten Vertretern unter den Viren, die mit dem Rinder-Grippe-Komplex in Zusammenhang stehen, gehören das Rinder-Herpes-Virus Typ I (BHV-1 oder infektiöses Rhinotracheitis Virus), das Parainfluenza Virus Typ 3 (PI-3), das die virale Rinder-Diarrhoe verursachende Virus (BVD) sowie das "Bovine Respiratory Syncytial Virus" (BRSV).

Viren können auf verschiedene Art und Weise eine Immunsuppression hervorrufen. Sie sind beispielsweise u.a. in der Lage, die Lymphozyten-Funktionen ganz oder teilweise auszuschalten oder die Aktivität körpereigener bakterizider Mechanismen herabzusetzen und zwar in erster Linie solche, die mit Makrophagen und Neutrophilen in Zusammenhang stehen, und die normalerweise dafür sorgen, dass Gram-negative Bakterien von den Lungen ferngehalten werden.

Die Viren verändern dabei die Fähigkeit der Makrophagen mit Antikörpern umgebene Bakterien zu verdauen (herabgesetzte $F_c$ Rezeptor Aktivität). Selbst wenn die Makrophagen noch in der Lage sind, die Bakterien zu inkorporieren, so ist dennoch die Abtötungseffizienz virusinfizierter Makrophagen deutlich herabgesetzt. Darüberhinaus konnte gezeigt werden, dass eine virale Infektion die Produktion chemotaktischer Substanzen durch die alveolaren Makrophagen stark reduzieren kann und damit die Erneuerung der Neutrophilen in der Lunge auf dramatische Weise beeinflusst.

Weiterhin kann eine Virus-Infektion die Mobilität der Neutrophilen verändern und zur Induktion von Fehlfunktionen führen.

Zuletzt kann die Funktionsfähigkeit der alveolären Makrophagen auch noch dadurch weiter beeinträchtigt werden, das sich cytotoxische T-Zellen gegen die Makrophagen-Zellen selbst richten, falls diese entweder aufgrund einer Replikation oder der Phagocytose von Viren, virale Antigene auf ihrer Oberfläche exprimieren.

Wie bereits zuvor erwähnt wurde, begünstigen virale Infektionen und Stress offenbar die Besiedlung des respiratorischen Traktes sowohl durch Gram-positive als auch durch Gram-negative Bakterien.

Obwohl eine Vielzahl von Bakterien existiert, die in der Lage sind, sich auf der Lunge anzusiedeln und Pneumonien zu verursachen, wird doch die Mehrzahl der Rinder Pneumonien durch Pasteurella haemolytica A-I hervorgerufen.

Darüberhinaus enthalten Isolate aus dem respiratorischen Trakt von Tieren, die an einer Erkrankung aus dem Bereich des Rinder-Grippe-Komplexes leiden, neben Pasteurella haemolytica oft auch Pasteurella multocida und Haemophilus somnus sowie verschiedene Mycoplasma-Spezies (Mycoplasma bovis, Mycoplasma dispar und Mycoplasma bovirhinis).

P. haemolytica beispielsweise kann nach seiner raschen Vermehrung im Nasopharinx-Bereich eingeschlossen in feinste Tröpfchen in die Lunge eindringen.

Darüberhinaus produziert P. haemolytica ein lösliches Cytotoxin (Leukotoxin), dass direkt die Funktion der Wiederkäuer-Leukocyten beeinträchtigen oder deren Abtötung verursachen kann.

Nach der Phagozytose der Bakterien können die alveolären Makrophagen und Neutrophilen dann aber geschädigt oder abgetötet werden.

Abgestorbene Neutrophile in der Lunge tragen mit zu den Läsionen bei, die durch eine Pneumonie hervorgerufen werden.

Die Krankheit bricht in der Regel innerhalb eines Zeitraums von 3 bis 15 Tagen aus, nachdem die Tiere starkem Stress ausgesetzt waren.

Die betroffenen Kälber sind in der Regel matt und appetitlos. Gewöhnlich tritt ein mucös-eitriger nasaler Ausfluss und ein oberflächlicher Husten auf. Die Respirationsrate steigt von einem Normalwert von 30 pro Minute auf Werte zwischen 80 bis 100 pro Minute an. Die erkrankten Kälber weisen darüberhinaus eine erhöhte Körpertemperatur im Bereich zwischen 40°C bis 41°C auf.

Eine Untersuchung der Lunge mit einem Stethoskop offenbart in der Regel Anzeichen einer Bronchopneumonie oder sogar einer Rippenfeltentzündung.

Pasteurella haemolytica ruft schwere Läsionen der Lunge hervor. Dagegen gehören Fibrin-Ablagerungen und kapillare Thrombosen zu den wesentlichen pathologischen Charakteristika von Pasteurellosen.

Die durch Erkrankungen aus dem Bereich des Rinder-Grippe-Komplexes hervorgerufenen Verluste der Rinder-Industrie sind nicht allein auf den Tod der betroffenen Tiere zurückzuführen, sondern ergeben sich auch aufgrund von Behandlungskosten, Gewichtsverlusten, uneffizienter Nahrungsverwertung sowie zusätzlicher Arbeitsstunden, die durch die Krankheit verursacht werden.

Es wurde daher auf der Suche nach adäquaten Behandlungsmethoden vorgeschlagen, das Auftreten der respiratorischen Erkrankungen bei Rindern sowie die Schwere des Krankheitsverlaufes durch Verabreichung von Interferonen einzudämmen.

Die Interferone (IFN) bilden eine Gruppe von Proteinen, die in drei Typen klassifiziert und mit $\alpha$, $\beta$ und $\gamma$ bezeichnet werden. Diese Einteilung in drei Gruppen basiert auf der antigenischen Struktur, der Gen-Organisation, der Struktur, der pH-Sensitivität, der zellulären Herkunft sowie auf der biologischen Aktivität der Interferon-Proteine.

Die verschiedenen Interferon-Typen können beispielsweise von Säugern, wie z.B. Rindern, Schweinen oder auch dem Menschen gewonnen werden.

Die Erwartung, dass es möglicherweise gelingen könnte, das Auftreten der respiratorischen Erkrankungen sowie die Schwere des Krankheitsverlaufes durch eine Behandlung mit Interferon zu begrenzen, konnte anhand von Modellversuchen erhärtet werden. In einer dieser Modell-Studien wurden Rinder arifiziell mit Herpes-Viren (BHV-1) infiziert. Die anschliessende Verabreichung von Human-Interferon brachte eine Reduktion in der Schwere des Infektionsverlaufes. Siehe Roney et al., Am.J.Vet.Res., 46: 1251-1255, 1985.

In vergleichbaren Modellstudien von Ohmann und Babiuk [J. Inf. Res. 5(4): 551-564 (1985)] wird die Wirkung von rekombinantem Rinder-Interferon auf die natürliche, zellulär-vermittelte Cytotoxizität untersucht. Zum Nachweis dieser Wirkung bedienen sich die Autoren sowohl eines *in vitro* [Behandlung von Leukozyten] als auch eines *in vivo* [Behandlung von artifiziell BHV-1 infizierten Kälbem] Test-Systems. In beiden Fällen konnte ein positiver Effekt auf die zellulär-vermittelte Cytotoxizität festgestellt werden.

Czerniecki *et al.* [Antiviral Res., Suppl. 1: 209-215 (1985)] beschreiben *in vitro* Versuche, die zeigen, dass BoIFN-$\alpha_l$1 in der Lage ist die Replikation verschiedener, mit BRD in Zusammenhang stehender Viren zu hemmen. Darüberhinaus werden *in vivo* Modell-Versuche präsentiert, die mit artifiziell infizierten [kombinierte BHV-1 und *Pasteurella haemolytica* Infektion] Kälbern durchgeführt wurden.

Babiuk *et al.* [J. Gen. Virol. 66: 2383-2394 (1985)] berichten über *in vivo* Experimente mit dem BHV-1/*Pasteurella haemolytica* Krankheitsmodell. Im Rahmen dieser Untersuchungen konnte erstmals eine prophylaktische Wirksamkeit von intranasal appliziertem Rinderinterferon gegen die Folgen einer artifiziellen Infektion mit einem bestimmten BHV-1 Virus-Stamm und einem definierten *Pasteurella haemolytica* Erreger im Rahmen und unter den spezifischen und standardisierten Bedingungen des Modells gezeigt werden.

Modellstudien sind jedoch in der Regel mit einem starken Fehler behaftet. So wurden im vorliegenden Fall beispielsweise die Rinder nicht einer Kombination von Stressfaktoren sowie einer Vielzahl verschiedener Viren und Mikroorganismen ausgesetzt, wie dies unter tatsächlichen Feldbedingungen der Fall ist, wo die Rinder durch Viehauktionen, Maststationen und durch Viehhöfe geschleust werden, was dazu beiträgt, dass sie sich in einem labilen physiologischen Zustand befinden, der u.a. durch eine vorübergehende Immunschwäche gekennzeichnet ist, wodurch diese Tiere besonders anfällig für Infektionskrankheiten sind.

Darüberhinaus handelt es sich bei den in den geschilderten Modell-Experimenten eingesetzten Rindern um gezielt ausgewählte, und für die im Rahmen des Modells verwendeten Pathogene seronegative Tiere, die erst nach der Interferonbehandlung gezielt und zu einem genau festgesetzten Zeitpunkt artifiziell mit einem bestimmten Virus, dem BHV-1 Virus, Stamm 108, infiziert werden. Nach einem Zeitraum von 4 Tagen folgt eine zweite Infektion mit einem ebenfalls definierten, superinfizierenden bakteriellen Pathogen [*Pasteurellla haemolytica,* Biotyp A, Serotyp 1].

Erfolgreiche Modellstudien bedeuten daher nicht notwendigerweise, dass Rinder auch unter Freilandbedingungen erfolgreich mit Rinder-Interferon praventiv gegen die Gesamtheit der unter dem Begriff des Rinder-Grippe-Komplexes zusammengefassten infektiosen respiratorischen Erkrankungen behandelt werden können. Darüberhinaus sind solchen Studien nur sehr beschränkt Angaben über die optimalen Rahmenbedingungen für eine Interferon-Behandlung zur Eindämmung infektiöser respiratorischer Erkrankungen sowie der Schwere des Krankheitsverlaufes unter Freilandbedingungen zu entnehmen.

Die Fragen nach dem besten Inokulations-Zeitpunkt sowie der günstigsten Inokulationsmethode bleiben daher weiterhin ungelöst.

Die Aufgabe, die es im Rahmen der Erfindung zu lösen galt, betrifft daher die Bereitstellung eines Arzneimittels, welches unter Praxisbedingungen zur prophylaktischen Behandlung von auf engem Raum gehaltenen, durch Stress, chemische Agentien und psychologische Faktoren für den sog. Rinder-Grippe-Komplex prädisponierten jungen Rindern, zur Verhinderung der unter diesem Begriff zusammengefassten Erkrankungen angewendet werden kann.

Wie aus der nun folgenden detaillierten Beschreibung der Erfindung ersichtlich wird, ist es im Rahmen dieser Erfindung überraschenderweise gelungen, diese und andere Aufgaben durch Verwendung eines rekombinanten Rinderinterferon-$\alpha$ zur Herstellung eines entsprechenden Arzneimittels zu lösen.

Die Erfindung betrifft somit in erster Linie die Verwendung eines rekombinanten Rinderinterferon-α zur Herstellung eines Arzneimittels zur einmaligen, intramuskulären, prophylaktischen Behandlung von auf engem Raum gehaltenen, durch Stress, chemische Agentien und psychologische Faktoren für den sog. Rinder-Grippe-Komplex prädisponierten jungen Rindern, zur Verhinderung dieser Erkrankung.

Besagtes Arzneimittel enthält das rekombinante Rinderinterferon-α vorzugsweise in einer Einzeldosis von 0.5 mg bis 250 mg, besonders bevorzugt in einer Einzeldosis von- 1.0 mg bis 50 mg und ganz besonders bevorzugt in einer Einzeldosis von 3.0 mg bis 10 mg.

Beschreibung der Erfindung

Interferone, die im Rahmen dieser Erfindung für die Herstellung eines Arzneimittels zur einmaligen, intramuskulären und prophylaktischen Behandlung von auf engem Raum gehaltenen, durch Stress, chemische Agentien und psychologische Faktoren für den sog. Rinder-Grippe-Komplex prädisponierten Rindern verwendet werden können, umfassen in erster Linie mit Hilfe der rekombinanten DNA Technologie hergestellte α-Interferone von Rindern.

Alle drei Interferon-Typen [α-, β-, und γ-] können eine unmittelbare Rolle bei der Kontrolle der Virus-Replikation in den betroffenen Zellen spielen. Sie sind ausserdem involviert in die Regulation der immunologischen Funktionen. Alle Interferone sind in der Lage, die erste Stufe der immunologiscben Abwehr - Phagocytose und Zellabtötung - zu stimulieren und zu erhöhen.

Im Rahmen der Erfindung werden Interferone vom α-Typ eingesetzt, die natürlicherweise von Lymphozyten und anderen Leukozyten als Antwort auf eine virale Infektion gebildet werden.

Die Klonierung und Expression von DNA-Fragmenten, die für Rinder-Interferon-α kodieren, hat gezeigt, dass das Rindergenom zwei verschiedene Klassen von α-Genen aufweist: Die BoIFN-α$_I$ und die BoIFN-α$_{II}$ Gene. Diese Gen-Klassen bestehen aus 10-12 bzw. 15-20 Subtypen. Das Gen, welches für BoIFN-α$_I$1, einen Subtyp aus der BoIFN-α$_I$ Familie, kodiert, wurde in Bakterienzellen kloniert und exprimiert, zur Bestimmung seiner in vitro und in vivo Aktivitäten.

Bei dem rekombinanten Rinder-Interferon-α$_I$ (rBoIFNα$_I$1) beispielsweise handelt es sich um ein basisches Protein, das aus 166 Aminosäuren zusammengesetzt ist. Das Molekulargewicht beträgt 19,000 Dalton.

Das formulierte Interferon wird gewöhnlich in einer lyophilisierten (gefriergetrockneten) Form aufbewahrt. Als Alternative können aber auch fertig-formulierte Injektionslösungen verwendet werden.

Das in lyophilisierter Form vorliegende Interferon kann durch Zugabe eines geeigneten Lösungsmittels, vorzugsweise von sterilem Wasser, wieder in eine für die Applikation geeignete Form überführt werden.

Die Administration des funktionsfähig wiederhergestellten Interferons erfolgt systemisch bei jungen Rindern und kann mit Hilfe an sich bekannter Verfahren, wie beispielsweise Injektion, durchgeführt werden. Eine systemische Applikation kann intramuskulär, vorzugsweise im Bereich des Rumpfes, subkutan oder intravenös erfolgen.

Besonders bevorzugt im Rahmen dieser Erfindung ist die Verwendung von rekombinantem Rinderinterferon-α zur Herstellung eines intramuskulär applizierbaren Arzneimittels zur einmaligen, prophylaktischen Behandlung von auf engem Raum gehaltenen, durch Stress, chemische Agentien und psychologische Faktoren für den sog. Rinder-Grippe-Komplex prädisponierten Rindern zur Verhinderung dieser Erkrankung.

Unter jungen Rindern sind erfindungsgemäss solche zu verstehen, deren Alter zwischen wenigen Tagen und 18 Monaten, insbesondere aber zwischen 6 und 18 Monaten liegt.

Die Menge des applizierten Interferons entspricht vorzugsweise einer prophylaktisch effektiven Dosis. Der untere Grenzwert liegt bei 0,5 mg IFN/Tier, vorzugsweise bei 1 mg IFN/Tier, insbesondere aber bei 3 mg IFN/Tier.

Der obere Grenzwert repräsentiert diejenige Maximalmenge an Interferon, die von dem behandelten Tier gerade noch ohne schwere Nebenwirkungen vertragen wird.

Der obere Grenzwert liegt beispielsweise bei 250 mg IFN/Tier, vorzugsweise bei 50 mg IFN/Tier und ganz besonders bevorzugt bei 10 mg IFN/Tier.

Die optimale Interferon-Menge liegt im Bereich von 3 mg IFN/Tier bis 10 mg IFN/Tier, insbesondere bei 5 mg IFN/Tier.

Das im Rahmen dieser Erfindung bereitgestellte Arzneimittel enthält somit das rekombinante Rinderinterferon-α vorzugsweise in einer Einzeldosis von 0.5 mg bis 250 mg, besonders bevorzugt in einer Einzeldosis von 1.0 mg bis 50 mg und ganz besonders bevorzugt in einer Einzeldosis von 3.0 mg bis 10 mg.

Das funktionsfähig wiederhergestellte Interferon kann in jeder geeigneten Konzentration vorliegen. So ist es beispielsweise möglich, für eine Injektion von 5 mg IFN/Tier eine IFN-Lösung von 2,5 mg IFN/ml Lösungsmittel (vorzugsweise steriles $H_2O$) herzustellen und 2 ml dieser Lösung pro Tier zu applizieren.

Wie aufgrund von pharmakokinetischen Studien festgestellt werden konnte, sind bereits nach 15-20 Stunden mehr als 90 % der applizierten Interferon-Menge eliminiert.

Die Interferon-Behandlung sollte daher vorzugsweise vor oder aber unmittelbar nach einer Exposition mit infektiösen, krankheitsverursachenden Mikroorganismen durchgeführt werden.

Der bevorzugte Zeitraum für die Administration des IFN umfasst eine Zeitspanne von -72 bis +24 Stunden, vorzugsweise von -60 bis +20 Stunden, bezogen auf den Zeitpunkt der Infektion mit einem infektiösen, krankheitserregenden Mikroorganismus.

Besonders bevorzugt ist eine Applikation vor dem Eintritt eines Infektionsereignisses, insbesondere ein Zeitraum von -50 bis -10 Stunden, bezogen auf den Zeitpunkt der Infektion mit einem infektiösen, krankheitserregenden Mikroorganismus.

Der exakte Zeitpunkt der Interferon-Applikation ist deshalb von entscheidender Bedeutung, da in der Mehrzahl der untersuchten Fälle die respiratorischen Erkrankungen entweder kurz nach dem Zusammenziehen der Tiere auf Rinderauktionen, während des Transports oder aber beim Eintritt in die Maststationen auftritt.

Es ist daher vorteilhaft, die Tiere dort bei der ersten möglichen Gelegenheit mit Interferon zu behandeln, wo sie vor der eigentlichen Mast zusammengezogen werden, i.e. auf Rinderauktionen, in Viehhöfen oder in Maststationen.

Die Wirksamkeit und Wirkungsweise des Interferon wurde im Rahmen dieser Erfindung sowohl in in vitro als auch in in vivo Experimenten demonstriert. Die klinische Effizienz einer Interferon-Applikation konnte dagegen sowohl in einer Modell-Studie, welche auf einem reproduzierbaren Krankheitsmodell beruht, als auch mit Hilfe von Freiland-Experimenten nachgewiesen werden.

Bei Freilandversuchen an insgesamt 3000 intranasal behandelten Kälbern und annähernd 7000 intramuskulär behandelten Tieren konnten keinerlei nachteilige Reaktionen, die in Zusammenhang mit der IFN-Behandlung stehen, beobachtet werden. Auch bei Tieren, die gleichzeitig mit der Interferon-Behandlung den üblichen Behandlungsprozeduren ausgesetzt waren, die routinemässig im Verlauf eines Mastbetrieb-Processings ablaufen, wie z.B. Wurmkuren, Impfungen, Ektoparasitizid-Behandlungen, das Implantieren von Anabolika, das Einbrennen von Brandzeichen sowie der Eintrag von Wachstums-Förderern und Antibiotika ins Mastfutter, konnten keinerlei nachteilige Effekte nachgewiesen werden.

In einer kontrollierten Studie konnte demonstriert werden, dass eine gleichzeitige IN oder IM Behandlung mit rekombinantem Rinderinterferon-$\alpha$ und eine Impfung mit modifizierten, lebenden IBR/PI-3 Virus-Vakzinen keinerlei ungünstige Effekte bei den behandelten Tieren hat und darüberhinaus die Schutzwirkung des Vakzines nicht beeinträchtigt wird.

Die Wirksamkeit der Interferon-Behandlung zur Eindämmung der respiratorischen Erkrankung bei Rindern sowie der Schwere des Krankheitsverlaufes, die im Rahmen dieser Erfindung in umfangreichen Modell- und Freiland-Studien demonstriert werden konnte, ergibt sich aus einer signifikanten Reduktion der Inzidenz und der Sterblichkeitsrate, einer signifikanten Reduktion des Schweregrades der Krankheit und der Anzahl der Krankheitstage pro Tier oder Gruppe sowie einer verbesserten Gewichtszunahme und Futterverwertung der behandelten Tiere im Vergleich zu den Kontrollen.

Die im folgenden aufgeführten Beispiele dienen nur der Erläuterung des zuvor beschriebenen Erfindungsgegenstands ohne diesen aber in irgendeiner Weise zu limitieren.

Ausführungsbeispiele:

1. Rinder-Interferon

Ein Verfahren zur Herstellung von Rinderinterferon ist in der Japanischen Patentanmeldung 58,224,690 sowie in der EP-A-0 088 622, die am 14. September 1983 unter dem Titel "Animal Interferons, Processes Involved In Their Production, Compositions Containing Them, DNA Sequences Coding Therefore and Expression Vehicles Containing Such Sequences and Cells Transformed Thereby" publiziert wurde, beschrieben.

2. Pharmakokinetik und Toxizität:

Die Verteilung von rBoIFN-$\alpha_I$ im Serum und dessen biologische Verfügbarkeit wurde an Kälbern nach intranasaler, intramuskulärer und intravenöser Applikation einer Einzeldosis von je 10 mg untersucht.

Die Halbwertszeiten bewegen sich zwischen 130 und 270 Minuten. Für alle Applikationsarten gilt, dass mehr als 90 % der total applizierten Dosis innerhalb von 15 Stunden nach der Applikation eliminiert sind und dass der Rinder-Interferon-Level nach 24 Stunden unter die Nachweisgrenze absinkt.

Von der intraumuskulär applizierten Dosis sind annähernd 40 % dem zirkulatorischen System zugänglich.

Bei Tieren, die an 15 aufeinanderfolgenden Tagen mit intranasal applizierten Dosen von bis zu 25 mg behandelt wurden, können keinerlei toxische Effekte des Interferons nachgewiesen werden. Am 16. Tag ermittelte Serum-Werte für Interferon liegen nur geringfügig über den Background-Werten.

Zuvor durchgeführte Untersuchungen haben ergeben, dass eine einzelne pro Kopf Dosis von 100 mg ohne weiteres toleriert wird.

Eine zu Beginn IM applizierte Anfangsdosis führt in vielen Fällen zu einer vorübergehenden milden Leukopenie und einer gemässigten fiebrigen Reaktion. Sowohl die Leukozyten-Zahlen als auch die Temperatur erreichen aber sehr schnell, spätestens jedoch innerhalb eines Zeitraumes von 24 Stunden bis 4 Tagen,, wieder Normalwerte. Eine täglich wiederholte Dosierung hat keinen weiteren Einfluss auf diese Depressionen, die den klinischen Zustand der Tiere offenbar in keiner Weise beeinflussen, ebensowenig wie die prophylaktische Wirkung des Interferons.

### 3. In vitro Untersuchungen der Interferon-Wirkung

a) Antivirale Aktivität in vitro:

Die antivirale Potenz des rBoIFN-$\alpha_I$1 wird mit Hilfe von Rinder-Zelllinien bestimmt, die mit verschiedenen rinderspezifischen Viren infiziert werden. Die Aktivitäts-Titer, die gewöhnlich als Einheit U/ml oder in umgerechneter Form als Einheit U/mg Protein (spezifische Aktivität) angegeben werden, geben den reziproken Wert derjenigen Verdünnung an, bei der eine 50 % Hemmung des virusinduzierten cytopathischen Effekts (Plaques) resultiert.

rBoIFN-$\alpha_I$1 hemmt die Virus-Replikation derjenigen Viren, die am häufigsten aus Rindern, die an "BRD" leiden, isoliert werden (BVDV, PI-3, BRVS und IBR/BHV-1).

b) Immunmodulatorische Effekte in vitro:

Zur Feststellung der immunmodulatorischen Effekte in vitro werden phagozytotisch aktive Rinderzellen (Neutrophile, Blut-Monocyten und alveoläre Makrophagen) mit rBoIFN-$\alpha_I$1 behandelt.

Die beobachteten Effekte sind sehr vielfältig und umfassen beispielsweise eine erhöhte Aufnahmerate für Bakterien für alle drei Zelltypen, eine gesteigete $F_c$-Rezeptor-Aktivität in alveolären Makrophagen, eine Hemmung sowohl der gerichteten als auch der ungerichteten Monocyten- und Polymorphen-Migration, eine gesteigerte Enzymfreisetzung oder Inaktivierung, eine gesteigerte Wasserstoffperoxid-Entwicklung sowie eine verminderte Peroxidamin-Freisetzung durch die alveolären Makrophagen und die PMN-Leukozyten.

Diese Effekte sind dosis- und zeitabhängig.

### 4. Immunmodulatorische Effekte *in vivo*

Im Verlauf einer experimentellen Misch-Infektion mit BHV-1/P. haemolytica, die durchgeführt wurde, um zu bestimmen, inwieweit Interferon auch in vivo eine Rolle als Immunmodulator spielt und damit zu einer Reduktion der Krankheits- und Todesfälle führt, wurden eine Vielzahl von Leukozytenfunktionen untersucht.

Im Modell erfolgt der Peak der Immunsuppression 4 Tage nach der erfolgten Injektion, zu einem Zeitpunkt also an dem die Empfänglichkeit für bakterielle Infektionen am grössten ist.

Um die Wirkung des rekombinanten Rinder-Interferons auf die Migration und chemotaktische Reaktion der Neutrophilen zu bestimmen, werden die Neutrophilen, die sich im peripheren Blut von Interferonbehandelten Tieren sowie von Kontrolltieren befinden, in einem Migrations- und Chemotaxis-Assay gemessen.

In beiden Fällen ist die Funktion der Neutrophilen infolge der Infektion supprimiert.

Die mit Interferon behandelte Gruppe erreicht aber sehr viel schneller wieder die Normalwerte als die Kontrollgruppe.

Eine weitere Veränderung in der Neutrophilen-Funktion kann mit Hilfe eines Chemiluminiszenz-Assays durch Bestimmung der Produktionsrate von reaktiven Sauerstoff-Abkömmlingen nachgewiesen werden. Mit Hilfe dieses Assays kann gezeigt werden, dass durch eine Interferon-Behandlung die Fähigkeit der Neutrophilen zur Herstellung von reaktiven Sauerstoff-Abkömmlingen in mit P. haemolytica injizierten Tieren dramatisch gesteigert ist.

## 5. Freiland-Versuche: "Shipping Fever"

In einer ersten Untersuchungsreihe wurden 770 eingekaufte Ochsen, auf Lastwagen zunächst zu einer Viehauktion und anschliessend zu einer Maststation transportiert. Bei der Ankunft wurden die Tiere zufallsgemäss auf identische Gehege verteilt. Jedes der Versuchstiere erhielt dann eine rBoIFN-$\alpha_i$1 Dosis von 5 mg pro Nüster in 2 ml Lösung oder eine äquivalente Menge eines Placebos.

Das Auftreten klinischer Symptome, wie z.B. Depressionen, Appetitlosigkeit oder respiratorische Symptome wird aufgezeichnet (siehe S.22). Kranke Tiere wurden mit Antibiotika behandelt.

Die Ergebnisse sind in der folgenden Tabelle wiedergegeben.

### Tabelle 1:

| Behandlungs-gruppe | Krankheits-häufigkeit[1]<br><br>(%, Tag 1-30) | Durchschnitt-liche Zahl der Krank-heitstage pro Tier | Futter-verwertung | Gewichts-zunahme<br><br>(kg/Tag) |
|---|---|---|---|---|
| Placebo | 38.2 | 0.956 | 6.6 | 0.91 |
| rBoIFN-$\alpha_I$1 | 31.9 | 0.839 | 6.0 | 1.0 |

[1] Anzahl kranker Tiere/Gesamtzahl der Tiere

Diese Freiland-Untersuchung zeigen, dass die Zahl der Tiere, die unter einer respiratorischen Erkrankung (Inzidenz) leiden sowie die Anzahl der Krankheitstage pro Tier bei den IFN behandelten Tieren signifikant reduziert sind im Vergleich zu den Kontrollen.

In analoger Weise sind bei den IFN-behandelten Tieren die Gewichtszunahme sowie die Futterverwertung deutlich besser.

In einer weiteren Studie, durchgeführt in einer Versuchs-Maststation, wurde die Wirksamkeit einer intramuskulär injizierten (10 mg IFN/Tier) oder durch intranasale Applikation (5 mg IFN/Nüster) verabreichten Einzeldosis mit der Wirksamkeit eines Placebos verglichen.

300 eingekaufte Kälber gleichen Geschlechts mit einem Gewicht im Bereich von 200 kg wurden durch eine kommerzielle Viehauktion geschleust, um eine adäquate Exposition mit einem potentiellen Infektionsherd für respiratorische Erkrankungen zu gewährleisten und anschliessend in LKW's verladen und auf das Versuchsgelände transportiert und zwar unter Bedingungen, die mit einer kommerziellen Verschickung vergleichbar sind.

Die Interferon-Applikation erfolgte auf der Viehauktion, 62-64 Stunden bevor die Tiere die Maststation erreichten.

In einer zweiten Studie wurde die Interferon-Applikation erst zum Zeitpunkt des Eintreffens der Tiere in der Maststation vorgenommen.

Dabei wurden in beiden Versuchsreihen die Anzahl kranker Tiere (in Prozent), die Anzahl der Krankheitstage sowie die Sterblichkeitsrate (in Prozent) für beide Applikationsarten (IM, IN) mit den Kontroll-Gruppen verglichen, die kein Interferon erhielten.

Die Ergebnisse sind in der folgenden Tabelle wiedergegeben:

Tabelle 2

| Experiment | Zeitpunkt der Applikation | Applikation | Anzahl Tiere | % krank | Zahl Krankheitstage[1] | % Sterblichkeit |
|---|---|---|---|---|---|---|
| 1 | Viehauktion (62-64 Std. vor Ankunft im Mastbetrieb) | IM | 107 | 44 | 2,02 | 1,9 |
|  |  | IN | 106 | 53 | 2,46 | 0,9 |
|  |  | Kontrolle | 106 | 63 | 2,79 | 5,7 |
| 2 | Bei Ankunft im Mastbetrieb | IM | 100 | 29 | 1,48 | 0 |
|  |  | IN | 99 | 28 | 1,61 | 0 |
|  |  | Kontrolle | 99 | 53 | 1,70 | 0 |

[1] Gesamtzahl der Krankheitstage/Anzahl Tiere, über einen Beobachtungszeitraum von 21 Tagen

Bei diesen Versuchen konnte nachgewiesen werden, dass sowohl IM als auch IN appliziertes rBoIFN-$\alpha_I$1 die Schwere und das Auftreten der BRD innerhalb der ersten drei Versuchswochen deutlich reduziert.

6. Freilandversuche "Enzootische Kälber-Pneumonie"

Auch die Wirksamkeit des rBoIFN-$\alpha_I$1 gegenüber der "Enzootischen Kälber-Pneumonie" wurde unter kommerziellen Bedingungen untersucht. 60-80 von verschiedenen Quellen eingekaufte hybride Simmentaler und Holsteiner Milch-Kälber, im Alter von 2-3 Wochen und mit einem Gewicht zwischen 50 bis 65 kg, wurden zu einer Viehauktion verbracht und anschliessend in die CG-eigene Versuchsstation in St.Aubin, Schweiz transportiert. Nur klinisch gesunde Tiere wurden für die Versuche zugelassen. Tiere, die vor Versuchsbeginn irgendwelche Anzeichen einer Erkrankung zeigten, wurden ausgesondert.

Nach der Ankunft wurden die Tiere gewogen, individuell markiert und gemäss einer geschichteten Zufallsverteilung (Geschlecht, Gewicht) auf 2 gleichartige Gehege pro Behandlungsgruppe innerhalb einer geschlossenen Stallung aufgeteilt.

Die Tiere wurden mit einem nicht-medikamentierten Milchersatz gefüttert. Unter diesen Bedingungen entwickelte ein grosser Prozentsatz (70 % bis 90 %) der Kälber spontan eine "BRD", innerhalb von 3-15 Tagen nach der Ankunft auf der Versuchsstation. Die im Zeitraum von 3 Wochen täglich durchgeführte klinische Untersuchung umfasst die folgenden Parameter:

Respirationsrate, Husten, nasaler Ausfluss, Temperatur, Appetit und allgemeines Verhalten (siehe S.22).

Tabelle 3

| Krankheitshäufigkeit | | | | |
|---|---|---|---|---|
| Untersuchung Nr. | Anzahl Kälber | Behandlung[2] | Dosis (mg) | Krankheitshäufigkeit[1] (%) |
| 1 | 58 | Kontrolle/$H_2O$ | - | 16/19 (85) |
|  |  | IFN d 0 (1x) | 0,5 | 11/18 (62) |
|  |  | IFN d 0 (1x) | 5,0 | 13/21 (62) |
| 2 | 59 | Kontrolle/$H_2O$ | - | 16/18 (90) |
|  |  | IFN d 0 (1x) | 5,0 | 16/20 (80) |
|  |  | IFN d 0,7,14 (3x) | 5,0 | 10/21 (48) |
| 3 | 78 | Kontrolle | - | 26/29 (89) |
|  |  | IFN d 0,4 (2x) | 5,0 | 20/22 (90) |
|  |  | IFN d 0 (1x) | 5,0 | 18/27 (67) |

[1] Innerhalb der Gruppen: Zahl kranker Tiere/Gesamtzahl der Tiere

[2] Intramuskuläre Injektion (2 ml Lösung) in die Nackenmuskulatur

d Tag(e) der Behandlung(en) bezogen auf die Einstellung (Tag 0)

Tabelle 4

| Sterblichkeit/Krankheitstage | | | | |
|---|---|---|---|---|
| Untersuchung Nr. | Anzahl Kälber | Behandlung | Dosis (mg) | Zahl der [1] Krankheitstage |
| 1 | 19 | Kontrolle/$H_2O$ | - | 1,84 |
| | 18 | IFN d 0 (1x) | 0,5 | 1,28 |
| | 21 | IFN d 0 (1x) | 5,0 | 1,14 |
| 2 | 18 | Kontrolle/$H_2O$ | - | 1,2 |
| | 20 | IFN d 0 (1x) | 5,0 | 1,1 |
| | 21 | IFN d 0,7,14 (3x) | 5,0 | 0,90 |
| 3 | 29 | Kontrolle | - | 1,34 |
| | 22 | IFN d 0,4 (2x) | 5,0 | 1,36 |
| | 27 | IFN d 0 (1x) | 5,0 | 1,00 |

[1] Innerhalb der Gruppen: Gesamtzahl der Krankheitstage/Anzahl Tiere, über einen Beobachtungszeitraum von 21 Tagen

d Tag(e) der Behandlung(en) bezogen auf die Einstallung (Tag 0)

Alle Versuchsgruppen zeigten deutlich bessere klinische Eigenschaften als die Placebo-Kontrollen. Die Krankheitshäufigkeit und die Anzahl der Krankheitstage pro Tier waren deutlich reduziert.

Die wöchentliche Verabreichung von rBoIFN-$\alpha_l$1 an den Tagen 0, 7 und 14 ergab eine Reduktion der Sterblichkeitsrate um bis zu 50 %.

Beschreibung des klinischen Bewertungssystems

Standardisierte klinische Untersuchung

Je nach Schweregrad der auftretenden Symptome werden 3 verschiedene Kategorien unterschieden, wobei der Normalzustand mit 1 und der schlechteste Zustand mit 3 bewertet wird.

| | | |
|---|---|---|
| Respirationsrate | 35 | 1 |
| | 35-60 | 2 |
| | >60 | 3 |
| Husten | kein Husten | 1 |
| | gelegentlich, schmerzlos, mild | 2 |
| | stark, schmerzhaft, häufig und Atemnot | 3 |
| Nasaler Ausfluss | keiner | 1 |
| | serös | 2 |
| | mucopurulent | 3 |
| Auskultation | normal | 1 |
| | verstärkte bronchiale Geräusche, wenige trockene oder feuchte Rasselgeräusche | 2 |
| | laute Rasselgeräusche und eine Vielzahl feuchter Rasselgeräusche und Anzeichen einer Pleuritis | 3 |
| Körpertemperatur | 39,5 °C | 1 |
| | 39,5-40,5 °C | 2 |
| | >40,5 °C | 3 |
| | Die Körpertemperatur wird 2x täglich gemessen und dann gemittelt | |
| Appetit | normal | 1 |
| | vermindert | 2 |
| | Anorexie | 3 |
| Verhalten | normal | 1 |
| | mässig gedämpft | 2 |
| | sehr stark gedämpft, moribund | 3 |

Interpretation der klinischen Bewertung

Zur Ermittlung eines Gesamtdurchschnittwertes [Overall Mean Value (OMV)] erhält jeder der oben im einzelnen aufgeführten Parameter einen bestimmten Koeffizienten.

| | |
|---|---|
| Respirationsrate | x 3 |
| Husten | x 1 |
| Nasaler Ausfluss | x 2 |
| Auskultation | x 2 |
| Körpertemperatur | x 3 |
| Appetit | x 3 |
| Verhalten | x 2 |

Gesamt  —

Gesamt/16 = OMV

Die Analyse der Krankheitsgeschichte kann aus der Entwicklung des Gesamtdurchschnittswertes (OMV), seiner Fläche unter der Kurve [area under the curve (AUC)] sowie der Ermittlung von AUC und OMV erhalten werden. Ein Vergleich zwischen den einzelnen Behandlungen erfolgt mit Hilfe der Kovarianz-Analyse.

**Patentansprüche**

1. Verwendung von rekombinantem Rinderinterferon-α zur Herstellung eines Arzneimittels zur einmaligen, intramuskulären, prophylaktischen Behandlung von auf engem Raum gehaltenen, durch Stress, chemische Agentien und psychologische Faktoren für den Rinder-Grippe-Komplex prädisponierten jungen Rindern, zur Verhinderung dieser Erkrankung.

2. Verwendung gemäss Anspruch 1, dadurch gekennzeichnet, dass das herzustellende Arzneimittel rekombinantes Rinderinterferon-α in einer Einzeldosis von 0.5 mg bis 250 mg enthält.

3. Verwendung gemäss Anspruch 2, dadurch gekennzeichnet, dass das herzustellende Arzneimittel rekombinantes Rinderinterferon-α in einer Einzeldosis von 1.0 mg bis 50 mg enthält.

4. Verwendung gemäss Anspruch 3, dadurch gekennzeichnet, dass das herzustellende Arzneimittel rekombinantes Rinderinterferon-α in einer Einzeldosis von 3.0 mg bis 10 mg enthält.

**Claims**

1. The use of recombinant bovine α-interferon for the preparation of a medicament for the single, intramuscular, prophylactic treatment of young cattle kept in close confinement and predisposed by stress, chemical agents and psychological factors to bovine respiratory disease complex, for the prevention of this disease.

2. The use according to claim 1, wherein the medicament to be prepared contains recombinant bovine α-interferon in a single dose of from 0.5 mg to 250 mg.

3. The use according to claim 2, wherein the medicament to be prepared contains recombinant bovine α-interferon in a single dose of from 1.0 mg to 50 mg.

4. The use according to claim 3, wherein the medicament to be prepared contains recombinant bovine α-interferon in a single dose of from 3.0 mg to 10 mg.

**Revendications**

1. Utilisation d'interféron α de bovin recombinant pour la préparation d'un médicament pour le traitement prophylactique intramusculaire en une fois de jeunes bovins maintenus dans un espace étroit, prédisposés au complexe grippal bovin par le stress, des agents chimiques et des facteurs psychologiques, afin d'inhiber cette maladie.

2. Application selon la revendication 1, caractérisée en ce que le médicament à préparer contient de l'interféron α de bovin recombinant en une dose unique de 0,5 mg à 250 mg.

3. Application selon la revendication 2, caractérisée en ce que le médicament à préparer contient de l'interféron α de bovin recombinant en une dose unique de 1,0 à 50 mg.

4. Application selon la revendication 3, caractérisée en ce que le médicament à préparer contient un interféron α de bovin recombinant en une dose unique de 3,0 mg à 10 mg.